# FASCICULE DE BREVET EUROPEEN

(11) **EP 4 081 507 B1**
(45) Date de publication et mention de la délivrance du brevet: **22.11.2023**
(21) Numéro de dépôt: 20851327.5
(22) Date de dépôt: 22.12.2020
(51) Int. Cl.: C07D 207/12, C07D 211/22, C07D 211/46, C07D 211/52, C07D 211/62, C07D 295/13, A61P 27/00, C07D 265/30, C07D 267/10, C07D 295/185, C07D 307/52, A61K 31/4453, A61K 31/451, A61K 31/445, A61K 31/341, A61K 31/5375, A61K 31/54, A61K 31/40, C07C 233/20, C07C 233/23

(54) **COMPOSÉS CHIMIQUES CIBLANT L'OEIL ET LEUR UTILISATION DANS LE TRAITEMENT DE MALADIES OCULAIRES**
AUF DAS AUGE ZIELENDE CHEMISCHE VERBINDUNGEN UND IHRE VERWENDUNG ZUR BEHANDLUNG VON AUGENERKRANKUNGEN
CHEMICAL COMPOUNDS TARGETING THE EYE AND USE THEREOF IN THE TREATMENT OF EYE DISEASES

(30) Priorité: 26.12.2019 FR 1915598
(43) Date de publication de la demande: 02.11.2022
(73) Titulaire: Biophytis, 75001 Paris (FR); Centre national de la recherche scientifique, 75016 Paris (FR); Institut National de la Santé et de la Recherche Médicale (INSERM), 75654 Paris Cedex 13 (FR); SORBONNE UNIVERSITE, 75006 Paris (FR)
(72) Inventeur: DINAN, Laurence, Crossways DORCHESTER, Dorset DT2 8BQ (GB); LAFONT, René, 75016 PARIS (FR); DILDA, Pierre, 75016 PARIS (FR); CAMELO, Serge, 93210 LA PLAINE SAINT-DENIS (FR); FONTAINE, Valérie, 77176 SAVIGNY LE TEMPLE (FR); BALDUCCI, Christine, 91180 SAINT GERMAIN LES ARPAJON (FR); MONTEIRO, Elodie, 94100 SAINT MAUR DES FOSSÉS (FR); GUIBOUT, Louis, 92600 ASNIÈRES-SUR-SEINE (FR); LATIL, Mathilde, 75019 PARIS (FR); SAHEL, José-Alain, 75013 PARIS (FR); VEILLET, Stanislas, 91600 SAVIGNY SUR ORGE (FR)
(74) Mandataire: Ipside
(86) Numéro de dépôt international: PCT/FR2020/052605
(87) Numéro de publication internationale: WO 2021/130451

(56) Documents cités:
- EP-A1- 0 030 009
- WO-A2-2004/011423
- JP-A- 2012 097 003
- FUHRHOP J-H ET AL: "BOLAFORM AMPHIPHILES WITH A RIGID HYDROPHOBIC BIXIN CORE IN SURFACEMONOLAYERS AND LIPID MEMBRANES", LANGMUIR, AMERICAN CHEMICAL SOCIETY, US, vol. 6, no. 2, 1 février 1990 (1990-02-01) , pages 497-505, XP000562590, ISSN: 0743-7463, DOI: 10.1021/LA00092A034
- TERUYUKI KOMATSU ET AL: "Solid Vesicle Membrane Made ofmeso-Tetrakis[(bixinylamino)-o-phenyl]po rphyrins", JOURNAL OF THE AMERICAN CHEMICAL SOCIETY, vol. 119, no. 48, 3 décembre 1997 (1997-12-03), pages 11660-11665, XP055735522, US ISSN: 0002-7863, DOI: 10.1021/ja971393d
- VALÉRIE FONTAINE ET AL: "Norbixin Protects Retinal Pigmented Epithelium Cells and Photoreceptors against A2E-Mediated Phototoxicity In Vitro and In Vivo", PLOS ONE, vol. 11, no. 12, 16 décembre 2016 (2016-12-16), page e0167793, XP055735506, DOI: 10.1371/journal.pone.0167793 cité dans la demande

## Description

### Domaine technique de l'invention

L'invention relève de composés chimiques néo-synthétisés ayant un tropisme pour l'œil après une administration par voie orale. Elle s'inscrit également dans le domaine des applications thérapeutiques et concerne l'utilisation desdits composés chimiques pour l'amélioration de la vision chez les mammifères.

Plus particulièrement, la présente invention vise l'utilisation de composés pour la protection des cellules de l'épithélium pigmentaire rétinien (EPR), notamment pour le traitement de la dégénérescence maculaire liée à l'âge (DMLA) ou encore de la maladie de Stargardt et la Rétinite pigmentaire chez les mammifères. L'invention a pour but d'améliorer la vision des individus atteints de ces maladies ou au moins de stabiliser l'évolution de ces maladies.

### Technique antérieure

La dégénérescence maculaire liée à l'âge (DMLA) est la cause la plus courante de cécité légale chez les populations de personnes âgées de 60 ans ou plus, en particulier en Europe et en Amérique du Nord (Smith et al. 2001). La DMLA affecte la partie centrale de la rétine, appelée macula, entraînant une grave déficience visuelle et la perte irréversible de la vision centrale.

La fonction maculaire est à l'origine de la vision centrale et de l'acuité visuelle dont la haute résolution est liée à sa forte concentration en photorécepteurs, spécifiquement responsables de la vision diurne et de la perception des couleurs, et appelés cônes. Le stade précoce de la DMLA est marqué par l'apparition de dépôts appelés Drüsen, et par une perte progressive des photorécepteurs spécialisés dans la vision nocturne appelées bâtonnets qui n'affectent la vision diurne que de façon marginale. Cependant, peu à peu, le sujet constate une altération de sa vision, qui est alors diagnostiquée par l'ophtalmologiste, c'est la forme intermédiaire ou maculopathie liée à l'âge (MLA). Les phases ultérieures de la DMLA peuvent prendre deux formes, la forme humide appelée aussi *exsudative* ou *néo-vasculaire* (caractérisée par la croissance de néo-vaisseaux choroïdiens dans l'espace sous-rétinien), ou l'*atrophie géographique* (forme sèche) qui se caractérise par la perte des cellules de l'épithélium pigmentaire rétinien (EPR) et des photorécepteurs qui sont les cellules nécessaires au cycle visuel. Le nombre de patients atteints de DMLA intermédiaire ou de la forme sèche est beaucoup plus important que le nombre de sujets atteints de la forme humide de la DMLA (Smith et al. 2001). Les dernières étapes de la DMLA, quelle que soit sa forme (humide ou sèche) conduisent à la destruction irréversible de la macula. L'évolution de la DMLA exsudative (forme humide) peut conduire à la cécité complète en quelques semaines alors que l'évolution de la DMLA sèche est généralement lente.

Bien que les mécanismes spécifiques impliqués dans l'initiation de la DMLA soient multiples il a été montré que le stress oxydatif et l'inflammation sont des éléments importants qui contribuent à sa physiopathologie. Les théories de l'étiologie de la DMLA incluent des modifications hydrodynamiques dans la membrane de Bruch causées par une accumulation progressive de matériel extracellulaire contenant des lipides, et la sénescence de l'EPR, dont l'activité est indispensable à la survie des photorécepteurs. Le vieillissement qui est le premier facteur de risque de la DMLA, provoque un dysfonctionnement des cellules de l'EPR et une insuffisance de leur métabolisme, ainsi que de leur activité phagocytaire. Une digestion incomplète des segments externes des photorécepteurs peut conduire à la formation de Drüsen qui réduisent la diffusion à travers la membrane de Bruch. Avec l'âge, l'EPR accumule une quantité croissante de lipofuscines. Celles-ci sont composées de lipides et de protéines, qui proviennent des phagolysosomes, des lysosomes présents dans les cellules de l'EPR et directement des photorécepteurs. Les lipofuscines contiennent également de la N-rétinyl-N-rétinylidène éthanolamine (A2E), qui est formée par la condensation de deux molécules de rétinaldéhyde avec une molécule d'éthanolamine.

Avec l'âge une accumulation accrue d'A2E dans la rétine des patients souffrant de MLA est observée (Bhosale et al., 2009). Sous l'action de la lumière bleue et en présence d'oxygène, l'A2E génère des espèces réactives qui provoquent des dommages aux protéines, aux lipides et à l'ADN, et donc un stress oxydatif important dans les cellules vieillissantes de l'EPR (Sparrow & Cai, 2001). Ces dommages perturbent l'activité lysosomiale des cellules de l'EPR et provoquent une accumulation de déchets, ce qui finit par engendrer, de place en place, la mort des cellules de l'EPR qui est suivie par celle des photorécepteurs auxquels elles étaient associées.

La maladie de Stargardt et la Rétinite pigmentaire sont des dégénérescences rétiniennes d'origines génétiques qui peuvent être causés par un nombre important de mutations ou de polymorphismes génétiques. Ces deux pathologies peuvent s'apparenter à la forme sèche de la DMLA puisqu'elles se caractérisent par une perte progressive des cellules de l'EPR et des photorécepteurs qui est liée à une accumulation progressive d'A2E dans le cas de la maladie de Stargardt. Comme pour la forme sèche de la DMLA, la dégénérescence des photorécepteurs et de l'EPR, conduisent à une perte de vision nocturne, puis tardivement de la vision centrale par les patients atteints par ces deux pathologies génétiques.

Parmi les traitements existants sont connues les injections intravitréennes d'anticorps anti-VEGF (Vascular Endothelial Growth Factor) qui permettent de bloquer partiellement la formation de néo-vaisseaux et offrent ainsi une option thérapeutique pour la forme humide de la DMLA. Il n'existe à l'heure actuelle aucun traitement sur le marché pour la forme sèche de la DMLA (MLA et atrophie géographique). De même, pour l'instant aucun médicament n'est disponible pour le traitement de la maladie de Stargardt ou de la Rétinite pigmentaire. Dans le cadre de la DMLA sèche des compléments alimentaires ont été formulés avec des composés antioxydants génériques, à savoir des minéraux et des vitamines aux propriétés antioxydantes, par exemple le zinc, les vitamines A, C, et E. Leur efficacité thérapeutique est réelle mais limitée à la MLA. Les formules AREDS nutraceutiques 1 et 2 (pour l'anglais « *Age-Related Eye Disease Study »,* AREDS 2001) sont considérées comme la norme de soins aux États-Unis pour le traitement de la MLA, réduisant le risque d'évolution vers la DMLA humide de 25 % et la perte de vision de 19 % sur cinq ans. De nombreux produits proposent une base de formulation commune : zinc et vitamines C et E, à laquelle sont ajoutés divers ingrédients : lutéine, resvératrol, acides gras oméga 3, sans toutefois disposer de données d'efficacité convaincantes sur ces ingrédients additionnels, ni sur les catégories de patients susceptibles de répondre favorablement à ces différentes molécules (Elliot & Williams, 2012).

Les caroténoïdes (des molécules exclusivement apportées par l'alimentation) ont été plus particulièrement étudiés, car certains d'entre eux (lutéine, zéaxanthine = des xanthophylles) sont naturellement présents dans la macula (Subczynski et al., 2010), et il est connu que ces composés possèdent un fort pouvoir antioxydant et absorbent également la lumière bleue limitant ainsi ses effets toxiques. C'est donc logiquement que ces composés ont été testés (seuls ou en combinaison) dans les formules AREDS. Les résultats obtenus ont été décevants, la supplémentation ne s'avérant efficace que pour un sous-ensemble de patients carencés en ces composés (Pinazo-Durán et al., 2014). D'autres xanthophylles ont également fait l'objet d'études par supplémentation orale, seules ou en combinaison avec la lutéine et/ou la zéaxanthine (ex. astaxanthine - Parisi et al., 2008). Des di-apo-caroténoïdes (= caroténoïdes tronqués aux deux extrémités - IUPAC chemical nomenclature) ont été testés *in vitro* et *in vivo,* en particulier la crocétine (= 8,8'-diapocarotene-8,8'-dioate) et ses glycosides (les crocines). Les crocines présentent un effet photo-protecteur *in vitro* sur des cultures primaires de photorécepteurs de bovins ou de primates (Laabich et al., 2006), et la crocétine protège les cellules neuro-ganglionnaires contre un stress oxydatif (Yamauchi et al., 2011). Des expérimentations ont également été réalisées avec un autre apo-caroténoïde, la bixine (= 6-methylhydrogen [9Z] 6,6'-diapocarotene-6,6'-dioate) ou certains de ses dérivés, *in vitro* sur des cellules neuro-ganglionnaires et *in vivo* par injections intravitréennes pour contrecarrer les effets d'un stress du réticulum endoplasmique (Tsuruma et al., 2012). Un extrait de graines d'Urucum (*Bixa orellana*) précédemment développé (Bixilia^{®}) enrichi en bixine a montré un effet photo-protecteur sur la peau humaine exposée aux UV (WO2010149942 ; Veillet et al., 2009) et sur des cellules d'EPR soumises à un stress photo-oxydant (WO2012156600 ; Fontaine et al., 2011). Enfin, la norbixine (6,6'-diapocarotene-6,6'-dioate) et en particulier sa forme 9'-cis, permet de diminuer fortement la mort cellulaire provoquée par une illumination avec des rayonnements bleus des cellules de l'EPR prétraitées avec de la N-rétinyl-N-rétinylidène éthanolamine (A2E) (WO2016174360 ; Lafont et al. 2015, Fontaine et al. 2016). Un traitement chronique par voie orale avec de la 9'-cis norbixine permet également de diminuer l'accumulation de A2E dans la rétine de souris double KO (ABCA4^{-/-}, RDH8^{-/-}) (Fontaine et al. 2016). Bien que la norbixine ait une bonne biodisponibilité son accumulation dans l'œil demeure très faible et de ce fait limite son activité protectrice de la rétine.

### Présentation de l'invention

Les inventeurs ont créé des composés innovants par hémisynthèse. Ils ont aussi découvert que certains de ces composés ont un meilleur profil pharmacocinétique et un meilleur tropisme pour l'œil que ceux de la norbixine. De plus, certains de ces composés ont une activité photo-protectrice de l'épithélium pigmentaire rétinien (EPR) équivalente ou supérieure à celle de la norbixine.

Selon un premier aspect, l'invention propose donc un composé chimique de formule générale **(I)** suivante : 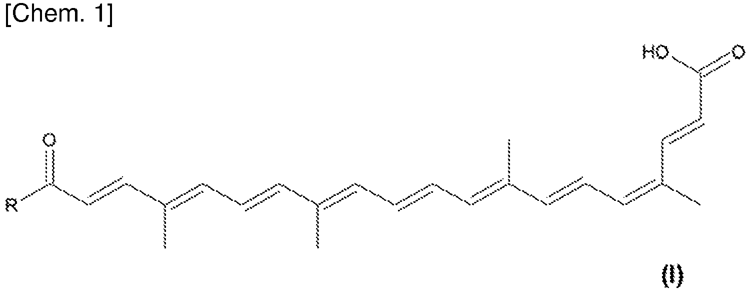
où COR est un amide secondaire ou tertiaire, tel que -R est choisi parmi :
   - -**M** ; -NH-(CH₂)ₙ-**M** et -NH-(CH₂)ₙ-C(CH₃)(CH₃)-**M**, -**M** étant choisi parmi
      a) 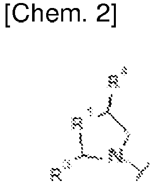
      b) 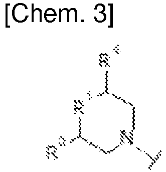
      c) 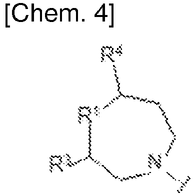 dans lesquels,
         - R¹ est choisi parmi un atome d'oxygène, un atome de soufre, un groupement >CH₂, >CH-O-(CH₂)ₙ-CH₃, >CH-(CH₂)ₙ-O-(CH₂)ₙ-CH₃, >CH-(CH₂)ₙ-OH, >CH-COOH, >C(OH)phényle ou >NH ;
         - R³ est choisi parmi un atome d'hydrogène, un groupement alkyle en Ci-Ce, -OH ou -O-alkyle en C₁-C₆ ;
         - R⁴ est choisi parmi un atome d'hydrogène, un groupement alkyle en Ci-Ce, -OH ou -O-alkyle en C₁-C₆ ;
         - n est un nombre entier compris entre 0 et 6 ;
   - -NH-(CH₂)ₙ-**W**, **W** étant un atome d'hydrogène, ou un groupement -OH, un groupement -O-(CH₂)ₙ-CH₃, ou un groupement choisi parmi
      i) 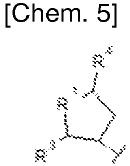
      ii) 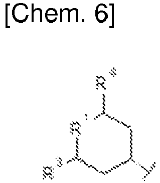
      iii) 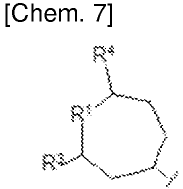
      iv) 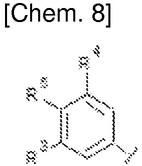
      v) 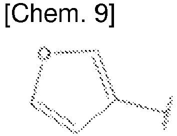 dans lesquels,
         - R¹ est choisi parmi un atome d'oxygène, un atome de soufre, un groupement >CH₂, >CH-O-(CH₂)ₙ-CH₃, >CH-(CH₂)ₙ-OH, >CH-COOH, >C(OH)phényle ou >NH ;
         - R³ est choisi parmi un atome d'hydrogène, un groupement alkyle en Ci-Ce, -OH ou -O-alkyle en C₁-C₆ ;
         - R⁴ est choisi parmi un atome d'hydrogène, un groupement alkyle en Ci-Ce, -OH ou -O-alkyle en C₁-C₆ ;
         - R⁵ est choisi parmi un groupement -CH₃, -OH, -O-(CH₂)ₙ-CH₃, - (CH₂)ₙ-OH ou -COOH ;
         - n est un nombre entier compris entre 0 et 6 ;
ainsi que les sels pharmaceutiquement acceptables dudit composé chimique.

Par composé chimique ont entend également les isomères dudit composé chimique et notamment les stéréoisomères.

Dans le cadre de la présente invention on entend par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

Dans le cadre de la présente invention on entend par « sels pharmaceutiquement acceptables d'un composé » des sels qui sont pharmaceutiquement acceptables, comme défini ici, et qui possèdent l'activité pharmacologique souhaitée du composé parent. De tels sels comprennent :
(1) les sels d'addition d'acide formés avec des acides minéraux tels que l'acide chlorhydrique, l'acide bromhydrique, l'acide sulfurique, l'acide nitrique, l'acide phosphorique et similaires ; ou formés avec des acides organiques tels que l'acide acétique, l'acide benzène-sulfonique, l'acide benzoïque, l'acide camphre-sulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires ; ou
(2) les sels formés lorsqu'un proton acide présent dans le composé parent soit est remplacé par un ion métallique, par exemple un ion de métal alcalin, un ion de métal alcalino-terreux ou un ion d'aluminium ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Selon un mode de réalisation particulier, l'invention vise un composé chimique de formule générale (I) choisi parmi les composés chimiques suivants :
- 1-[2-méthoxyéthanamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[1,4-oxazinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[pipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[2-hydoxyéthanamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1[1,4-oxazépanamido](2*E*,4*E*,6*E*,8*E*,10*E*,12*E*,14*E*,16*Z*,18*E*)-4,8,13,17-tétramethylicosa-2,4,6,8,10,12,14,16,18-nonaènedioate ;
- 1-thiomorpholinamido-(2*E*,4*E*,6*E*,8*E*,10*E*,12*E*,14*E*,16*Z*,18*E*)-4,8,13,17-tétramethylicosa-2,4,6,8,10,12,14,16,18-nonaènedioate ;
- 1-pyrrolidinamido-(2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[2-morpholinopropanamido](2*E*,4*E*,6*E*,8*E*,10*E*,12*E*,14*E*,16*Z*,18*E*)-4,8,13,17-tétramethylicosa-2,4,6,8,10,12,14,16,18-nonaènedioate ;
- 1-[(*S*)-3-hydroxypyrrolidinamido](2*E*,4*E*,6*E*,8*E*,10*E*,12*E*,14*E*,16*Z*,18*E*)-4,8,13,17-tétramethylicosa-2,4,6,8,10,12,14,16,18-nonaènedioate ;
- 1-[2-morpholinoethanamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[(*R*)-3-hydroxypyrrolidinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[4-hydroxypipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[2-methyl-2-(4-morpholinyl)propylamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[4-hydroxyméthylpipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[(Z)-2,6-diméthylmorpholinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[4-hydroxyphénylamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[4-benzyl-4-hydroxypipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[4-carboxypipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[(E)-4-hydroxycyclohexamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[3-méthoxypipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[4-méthoxypipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[2-(2-furyl)éthanamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[4-n-propoxypipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[4-éthylméthoxypipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate.

Selon un deuxième aspect, la présente invention vise une composition comportant au moins un composé chimique objet de la présente invention.

Selon un mode de réalisation particulier de la présente invention, la composition comprend au moins un excipient. Des exemples d'excipients sont des charges, des diluants, des désintégrants, des lubrifiants, des conservateurs, des délitants, des édulcorant, etc. Selon un exemple particulier, au moins un excipient est choisi parmi une huile, de l'eau et un alcool, un silicone.

Selon un mode de réalisation particulier de la présente invention, la composition comprend un support sous une forme adaptée pour être ingéré, injecté dans l'œil ou injecté dans le sang

Selon un troisième aspect la présente invention vise un composé chimique de formule générale (I) ou la composition comportant au moins un composé chimique de formule générale (I) pour son utilisation en tant que médicament.

Selon un mode de réalisation l'invention vise le composé chimique de formule générale (I) ou la composition comportant au moins un composé chimique de formule générale (I) pour son utilisation pour le traitement et/ou la prévention des dommages à la rétine des mammifères causés par des dégénérescences rétiniennes.

Selon un mode de réalisation l'invention vise le composé chimique de formule générale (I) ou la composition comportant au moins un composé chimique de formule générale (I) pour son utilisation pour la photoprotection des cellules de l'épithélium pigmentaire rétinien chez les mammifères.

Selon un mode de réalisation l'invention vise le composé chimique de formule générale (I) ou la composition comportant au moins un composé chimique de formule générale (I) pour son utilisation pour la prévention des dommages à la rétine des mammifères causés par l'exposition aux rayonnements bleus correspondant à la bande bleue du spectre de la lumière visible, de longueur d'onde comprise entre 435 nm et 490 nm.

Selon un mode de réalisation l'invention vise le composé chimique de formule générale (I) ou la composition comportant au moins un composé chimique de formule générale (I) pour son utilisation dans le traitement et/ou la prévention des maladies oculaires chez les mammifères.

Selon un mode de réalisation l'invention vise le composé chimique de formule générale (I) ou la composition comportant au moins un composé chimique de formule générale (I) pour son utilisation dans le traitement et/ou la prévention des rétinopathies chez les mammifères.

Selon un mode de réalisation l'invention vise le composé chimique de formule générale (I) ou la composition comportant au moins un composé chimique de formule générale (I) pour son utilisation dans le traitement et/ou la prévention de la dégénérescence maculaire liée à l'âge (DMLA) chez les mammifères. Le composé chimique objet de la présente invention permet avantageusement d'améliorer la vision des individus atteints de cette maladie ou au moins de stabiliser l'évolution de la maladie.

Selon un autre mode de réalisation l'invention vise le composé chimique de formule générale (I) ou la composition comportant au moins un composé chimique de formule générale (I) pour son utilisation dans le traitement et/ou la prévention de la maladie de Stargardt et la Rétinite pigmentaire chez les mammifères. Le composé chimique objet de la présente invention permet avantageusement d'améliorer la vision des individus atteints de ces maladies.

### Brève description des figures

L'invention sera mieux comprise à la lecture de la description suivante, donnée à titre d'exemple nullement limitatif, et faite en se référant aux figures qui représentent :

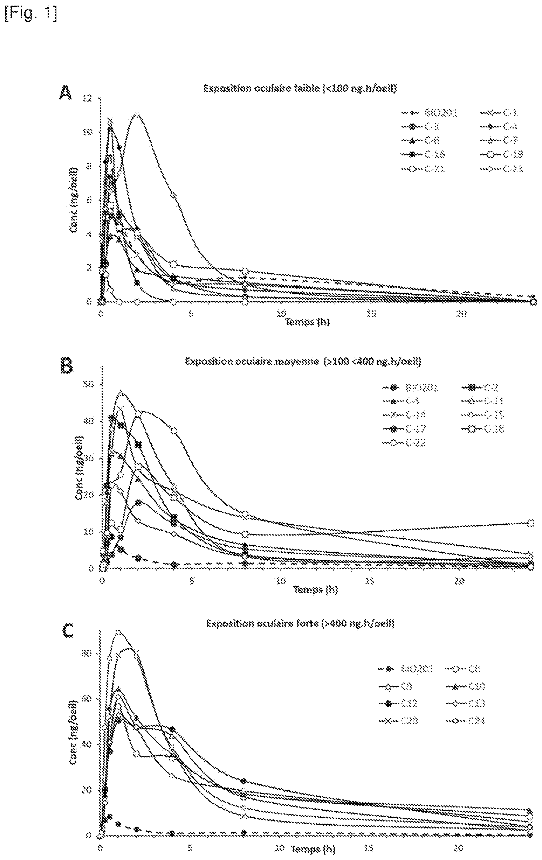

[Fig. 1] La figure 1 représente des profils pharmacocinétiques de composés objet de la présente invention dans l'œil. Il s'agit de graphiques représentant les concentrations oculaires en fonction du temps après administration des Composés chimiques (C) objet de la présente invention et de la norbixine (BIO201). Tous les composés ont été administrés par voie orale (p.o.) à 50 mg/kg formulés en milieu D-α-Tocopheryl polyethylene glycol 1000 succinate (VitE-TPGS) 20% en tampon bicarbonate de sodium (0.1M). Les profils pharmacocinétiques des composés ayant une faible exposition oculaire sont représentés dans le graphique A de la figure 1, ceux ayant une exposition oculaire moyenne sont représentés dans le graphique B de la figure 1, et ceux ayant une exposition oculaire forte sont représentés dans le graphique C de la figure 1.

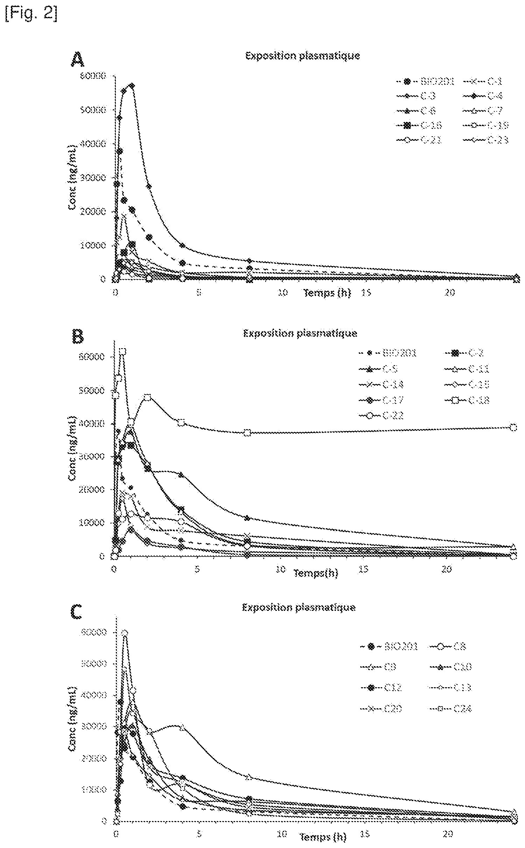

[Fig. 2] La figure 2 représente des profils pharmacocinétiques de composés objet de la présente invention dans le plasma. Il s'agit de graphiques représentant les concentrations plasmatiques en fonction du temps après administration des Composés (C) et de la norbixine (BIO201). Tous les composés ont été administrés par voie orale (p.o.) à 50 mg/kg formulés en milieu VitE-TPGS 20% en tampon bicarbonate de sodium (0.1M). Les profils d'exposition plasmatique des composés ayant une faible exposition oculaire sont représentés dans le graphique A de la figure 2, ceux des composés ayant une exposition oculaire moyenne sont représentés dans le graphique B de la figure 2, et enfin ceux des composés ayant une exposition oculaire forte sont représentés dans le graphique C de la figure 2.

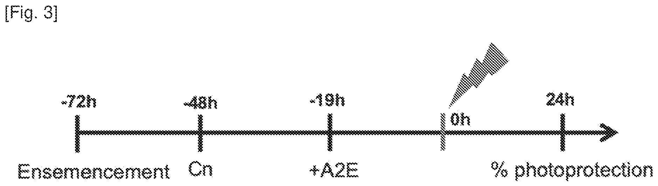

[Fig. 3] La figure 3 représente un chronogramme des expériences de photoprotection des cellules de l'épithélium pigmentaire rétinien (EPR) par des composés (C) objet de la présente invention *in vitro.* Il s'agit de la chronologie des étapes mises en oeuvre afin de tester l'activité photo-protectrice des différents composés chimiques (C) comparée à celle de la norbixine native sur les cellules de l'EPR mises en présence d'A2E et soumises à une illumination.

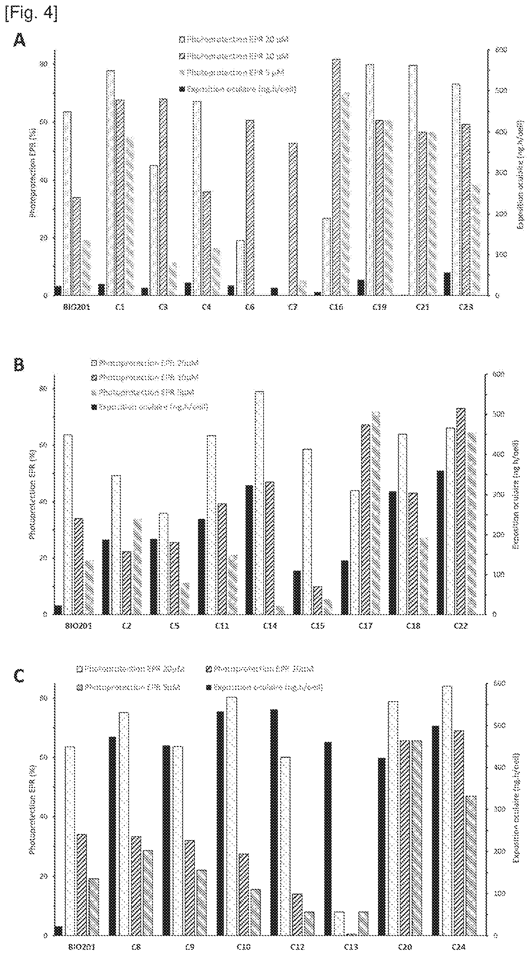

[Fig. 4] La figure 4 représente le ciblage de l'œil et le pourcentage de photoprotection *in vitro* des composés objet de la présente invention. Il s'agit d'histogrammes présentant de manière combinée les aires sous la courbe des concentrations oculaires (exposition oculaire) de la norbixine (BIO201) et des différents composés C objet de la présente invention, et les pourcentages de photoprotection obtenus avec la norbixine et les composés objet de la présente invention à 5µM, 10µM et 20µM. Les composés ayant une faible exposition oculaire sont représentés dans l'histogramme A, ceux ayant une exposition oculaire moyenne sont représentés dans l'histogramme B, et ceux ayant une forte exposition oculaire sont représentés dans l'histogramme C.

### Description des modes de réalisation

Les composés chimiques objet de la présente invention sont inexistants dans les bases de données chimiques des caroténoïdes et di-apo-caroténoïdes. Ils sont synthétisés selon des processus industrialisables, c'est-à-dire avec un minimum d'étapes de synthèse et un rendement optimal. Ils ont un meilleur profil pharmacocinétique et un meilleur tropisme pour l'œil que ceux de la norbixine. Certains de ces composés ont par ailleurs une activité photo-protectrice de l'EPR supérieure à celle de la norbixine.

### DESCRIPTION DES SYNTHESES ET SCHEMAS GENERAUX

Les composés chimiques de formule générale (I) : 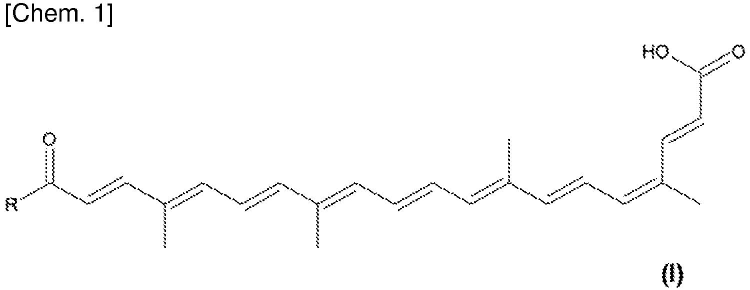 peuvent être préparés par application ou adaptation de toute méthode connue en soi de l'homme du métier et/ou à la portée de ce dernier, qui ont pu être décrites, ou par application ou adaptation des procédés décrits dans les procédures qui suivent.

Dans la description qui suit les différents groupements font référence aux définitions précédemment données, c'est-à-dire :
COR est un amide secondaire ou tertiaire, tel que -R est choisi parmi :
- -**M** ; -NH-(CH₂)ₙ-**M** et -NH-(CH₂)ₙ-C(CH₃)(CH₃)-**M**, -**M** étant choisi parmi
   a) 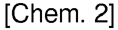 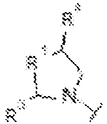
   b) 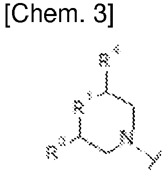
   c) 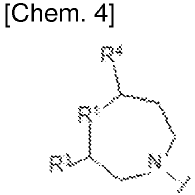 dans lesquels,
      - R¹ est choisi parmi un atome d'oxygène, un atome de soufre, un groupement >CH₂, >CH-O-(CH₂)ₙ-CH₃, >CH-(CH₂)ₙ-O-(CH₂)ₙ-CH₃, >CH-(CH₂)ₙ-OH, >CH-COOH, >C(OH)phényle ou >NH ;
      - R³ est choisi parmi un atome d'hydrogène, un groupement alkyle en Ci-Ce, -OH ou -O-alkyle en C₁-C₆ ;
      - R⁴ est choisi parmi un atome d'hydrogène, un groupement alkyle en Ci-Ce, -OH ou -O-alkyle en C₁-C₆ ;
      - n est un nombre entier compris entre 0 et 6 ;
- -NH-(CH₂)ₙ-**W**, **W** étant un atome d'hydrogène, ou un groupement -OH, un groupement -O-(CH₂)ₙ-CH₃, ou un groupement choisi parmi
   i) 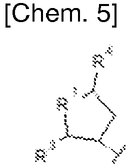
   ii) 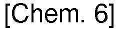 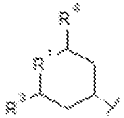
   iii) 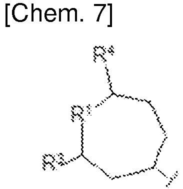
   iv) 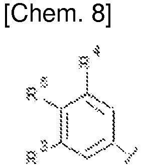
   v) 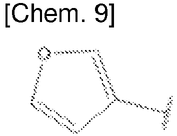 dans lesquels,
      - R¹ est choisi parmi un atome d'oxygène, un atome de soufre, un groupement >CH₂, >CH-O-(CH₂)ₙ-CH₃, >CH-(CH₂)ₙ-OH, >CH-COOH, >C(OH)phényle ou >NH ;
      - R³ est choisi parmi un atome d'hydrogène, un groupement alkyle en Ci-Ce, -OH ou -O-alkyle en C₁-C₆ ;
      - R⁴ est choisi parmi un atome d'hydrogène, un groupement alkyle en Ci-Ce, -OH ou -O-alkyle en C₁-C₆ ;
      - R⁵ est choisi parmi un groupement -CH₃, -OH, -O-(CH₂)ₙ-CH₃, - (CH₂)ₙ-OH ou -COOH ;
      - n est un nombre entier compris entre 0 et 6.

Dans le cadre de la présente invention on entend par « groupe alkyle en C₁-C₆ », tout groupe alkyle de 1 à 6 atomes de carbones, linéaire ou ramifié, en particulier, les groupes méthyle, éthyle, n-propyle, iso-propyle, n-butyle, iso-butyle, sec-butyle, t-butyle, n-pentyle, n-hexyle. Avantageusement il s'agit d'un groupe méthyle, éthyle, iso-propyle ou t-butyle, en particulier d'un groupe méthyle ou éthyle, plus particulièrement d'un groupe méthyle.

**Schéma A** pour la synthèse du composé 1-[2-(2-furyl)éthanamido](2*E*,4*E*,6*E*,8*E*,10*E*,12*E*,14*E*,16*Z*,18*E*)-4,8,13,17-tétraméthylicosa-2,4,6,8,10,12,14,16,18-nonaenedioate (appelé composé C21) :
Formation de l'amide (première méthode)

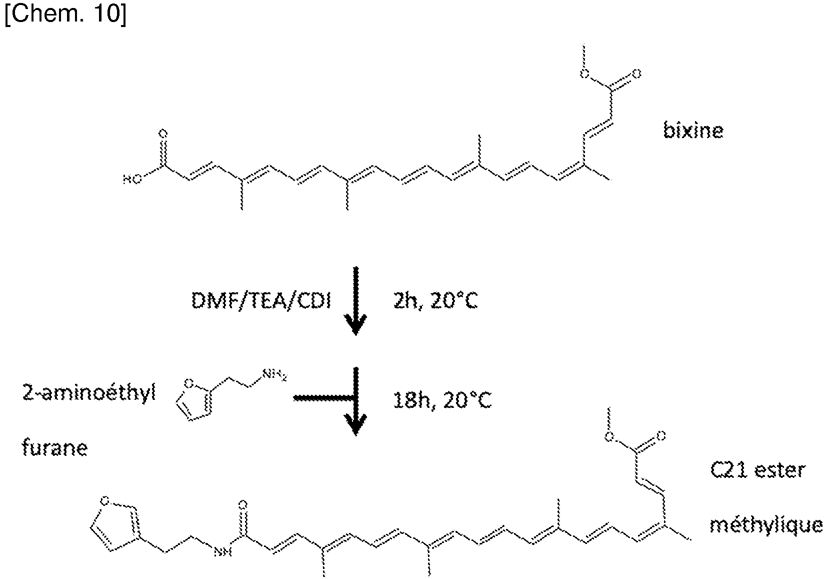

### L'hydrolyse de l'ester méthylique

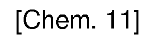

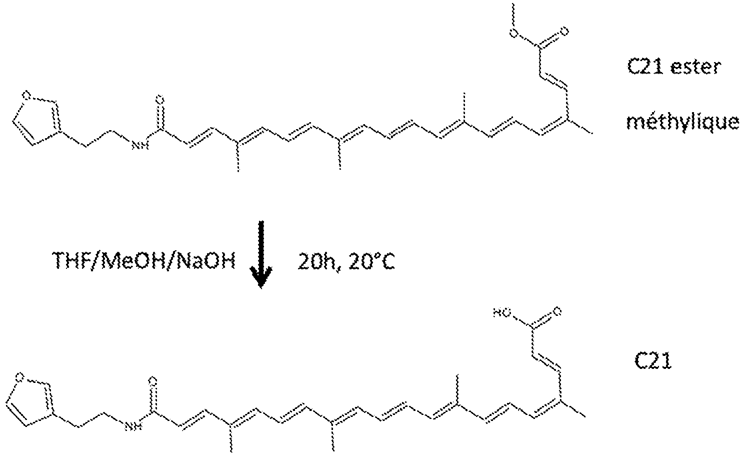

**Schéma B** pour la synthèse du composé 1-[(E)-4-hydroxycyclohexamido](2*E*_{,}4*E*,6*E*,8*E*,10*E*,12*E*,14*E*,16*Z*,18*E)*-4,8,13,17-tétraméthylicosa-2,4,6,8,10,12,14,16,18-nonaènedioate (appelé composé C19 :
Formation de l'amide (seconde méthode) 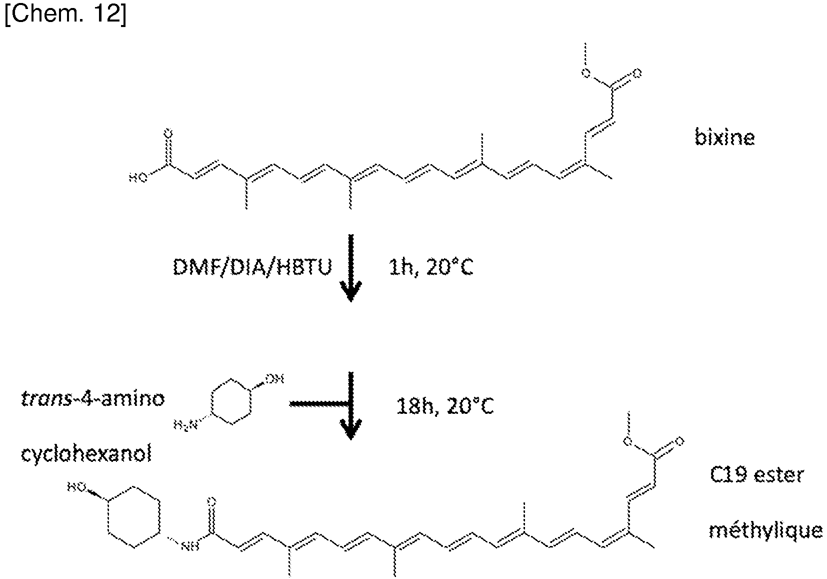
DMF = diméthylformamide
TEA = triéthylamine
DIA = diisopropylamine
THF = tétrahydrofurane
CDI = carbonyldiimidazole
HBTU = hexafluorophosphate de (1H-benzotriazol-1-yloxy)(diméthylamino)-N,N-diméthylméthaniminium

### Exemples :

### Matériels et méthodes

Les spectres de résonance magnétique nucléaire (RMN) du proton (¹H) sont effectués sur un appareil Bruker Avance DPX500 (500, 0.7 MHz). Les déplacements chimiques (δ) sont mesurés en partie par million (ppm). Les spectres sont calibrés sur le déplacement chimique du solvant deutéré utilisé. Les constantes de couplage (J) sont exprimées en Hertz (Hz) et la multiplicité est représentée de la manière suivante, singulet (s), doublet (d), doublet de doublet (dd), triplet (t), triplet de doublet (td), quadruplet (q), multiplet (m). Les spectres de masse (SM) sont réalisés avec un spectromètre Agilent Technologies MSD, type G1946A, les échantillons sont ionisés par une source « Atmospheric pressure chemical ionization » (APCI).

A titre d'exemples illustratifs de l'invention, les molécules représentées dans le tableau 1 ont été synthétisées.

**[Tableau 1]**

| **N°** | **Structure chimique** | **Nom chimique** |
|---|---|---|
| **C1** | 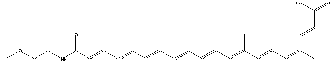 | 1-[2-méthoxyéthanamido] (2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C2** | 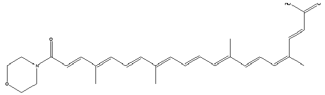 | 1-[1,4-oxazinamido] (2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C3** | 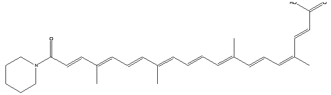 | 1-[pipéridinamido](2*E*,4*E*,6*E*,8*E*,10*E*, 12*E*,14*E*,16*Z*,18*E*)-4,S,13,17-tétraméthylicosa-2,4,6,8,10,12,14,16,18-nonaènedioate |
| **C4** | 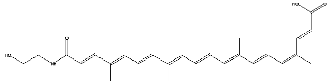 | 1-[2-hydroxyéthanamido] (2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C5** | 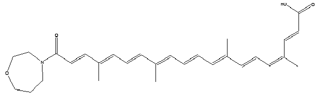 | 1[1,4-oxazépanamido] (2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C6** | 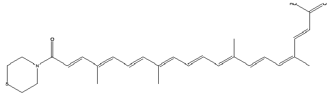 | 1-thiomorpholinamido-(2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C7** | 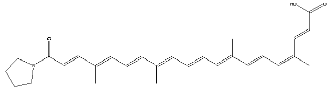 | 1-pyrrolidinamido-(2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C8** | 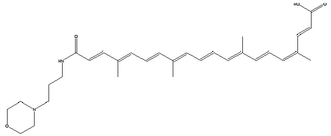 | 1-[2-morpholinopropanamido] (2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C9** | 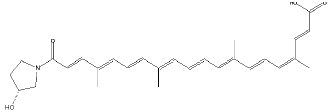 | 1-[(*S*)-3-hydroxypyrrolidinamido] (2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C10** | 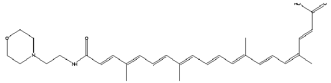 | 1-[2-morpholinoéthanamido] (2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C11** | 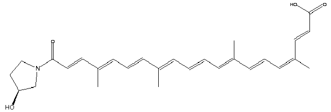 | 1-[(*R*)-3-hydroxypyrrolidinamido] (2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C12** | 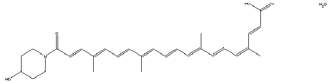 | 1-[4-hydroxypipéridinamido] (2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C13** | 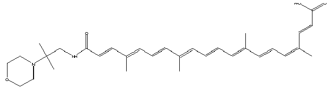 | 1-[2-méthyl-2-(4-morpholinyl) propylamido] (2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C14** | 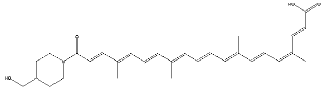 | 1-[4-hydroxyméthylpipéridinamido] (2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C15** | 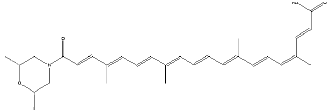 | 1-[(Z)-3,5-diméthylmorpholinamido](2*E*,4*E*,6*E* ,8*E*,10*E*,12*E,*14E,16*Z*,18*E*)-4,8,13,17-tétraméthylicosa-2,4,6,8,10,12,14,16,18-nonaènedioate |
| **C16** | 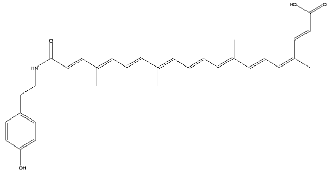 | 1-[4-hydroxyphénylethanamido] (2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C17** | 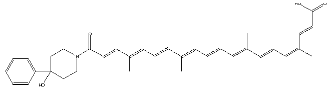 | 1-[4-benzyl-4-hydroxypipéridinamido] (2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C18** | 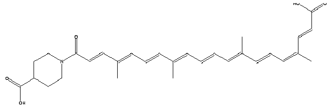 | 1-[4-carboxypipéridinamido] (2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C19** | 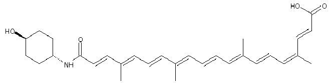 | 1-[(E)-4-hydroxycyclohexamido] (2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C20** | 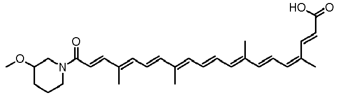 | 1-[3-méthoxypiperidinamido] (2*E*,4*E*,6*E*,8*E*,10*E*,12*E*,14*E*,16*Z*,18 *E*)-4,8,13,17-tétraméthylicosa-2,4,6,8,10,12,14,16,18-nonaènedioate |
| **C21** | 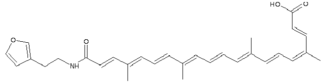 | 1-[2-(2-furyl) éthanamido](2*E*,4*E*,6*E*,8*E*,10*E*,12*E* ,14*E*,16*Z*,18*E*)-4,8,13,17-tétraméthylicosa-2,4,6,8,10,12,14,16,18-nonaènedioate |
| **C22** | 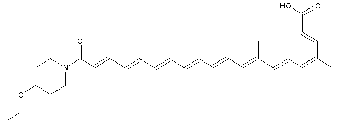 | 1-[4-n-propoxypipéridinamido](2*E*,4*E*,6*E*, 8*E*,10*E*,12*E*,14*E*,16*Z*,18*E*)-4,8,13,17-tétraméthylicosa-2,4,6,8,10,12,14,16,18-nonaènedioate |
| **C23** | 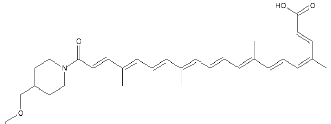 | 1-[4-éthylméthoxypipéridinamido](2*E*,4 *E*,6*E*,8*E*,10*E*,12*E*,14*E*,16*Z*,18*E*)-4,8,13,17-tétraméthylicosa-2,4,6,8,10,12,14,16,18-nonaènedioate |
| **C24** | 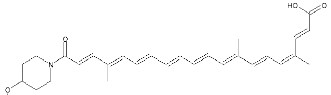 | 1-[4-méthoxypipéridinamido](2*E*,4*E*,6*E*, 8*E*,10*E*,12*E*,14*E*,16*Z*,18*E*)-4,8,13,17-tétraméthylicosa-2,4,6,8,10,12,14,16,18-nonaènedioate |

### Exemple 1 (Schéma A)

### Préparation du composé C21

### Etape 1 (Méthode type de couplage d'une amine au carbonyldiimidazole (CDI)) : préparation de l'ester méthylique de C21

Les réactions chimiques ont été réalisées sous atmosphère d'argon. Les réactifs et les produits ont été protégés de la lumière par du papier d'aluminium pendant toutes les étapes de la réaction et lors de l'étape d'extraction et d'élimination des solvants. Les produits ont été conservés à 4°C. La bixine (1g ; 2.54 mmol) est dissoute dans 10 mL de diméthylformamide (DMF) sec et de la triéthylamine (1.06 mL; 7.61 mmol) puis du CDI (0.823 g; 5.07 mmol) sont rajoutés. Au bout de deux heures, du 2-aminoéthylfurane (845 mg ; 7.61 mmol) a été ajouté et le mélange réactionnel a été agité pendant 18 h à 20°C. De l'acide chlorhydrique (HCl, 1 N ; 20 mL) est rajouté et la suspension résultante a été centrifugée. Suite à l'élimination du surnageant, le culot contenant l'ester méthylique a été re-suspendu deux fois en présence d'eau. Suite à une nouvelle centrifugation, le culot (1.24 g) a été utilisé directement dans l'étape d'hydrolyse.

### Etape 2 (Procédure type de l'hydrolyse) : conversion de l'ester méthylique de C21 en C21

Les réactions chimiques ont été réalisées sous atmosphère d'argon et protégées de la lumière par du papier d'aluminium. Les produits sont stockés à 4°C. Le mèthyl ester de C-21 est dissout dans 15 mL de tétrahydrofurane (THF) et 13 mL de MeOH. De la soude (NaOH, 1 N; 15.3 mL) est ajoutée et le mélange réactionnel est agité pendant 20 h à 20°C. De l'acide chlorhydrique (HCl ; 1 N; 16 mL) a été ajouté progressivement. La suspension obtenue est centrifugée et le surnageant est éliminé. Le culot est ensuite re-suspendu et mélangé deux fois avec de l'eau puis centrifugé à nouveau et le surnageant est éliminé. La pâte obtenue, contenant de l'eau, est transférée dans un ballon pyriforme en présence d'eau et d'acétonitrile, et lyophilisée pour donner 1.04 g d'une poudre de couleur orangée à rouge.

### Analyses du composé C21

LC-MS : m/z = 474.3 (MH⁺) pureté UV à 460 nm = 99%.

RMN ¹H (500 MHz, DMSO-*d₆*) - δ 8.11 (t, 1H), 7.54 (s, 1H), 6.19 (d, 1H), 3.42-3.38 (m, 2H), 2.78 (t, 2H).

### Préparation du composé C19

### Etape 1 (Procédure type pour le couplage de l'amine en utilisant le HBTU) : préparation de l'ester méthylique de C19 (méthode utilisée pour la préparation de l'ester méthylique de C19 uniquement, la réaction utilisant le CDI étant trop lente pour ce composé)

Les réactions chimiques ont été réalisées sous atmosphère d'argon. Les réactifs et les produits ont été protégés de la lumière par du papier d'aluminium pendant toutes les étapes de la réaction et lors de l'étape d'extraction et d'élimination des solvants. Tous les produits sont conservés à 4°C. La bixine (1.17 g ; 2.97 mmol) est dissoute dans 20 mL de DMF anhydre et de diisopropylamine (1.23 mL ; 7.42 mmol) ; on ajoute ensuite le HBTU (hexafluorophosphate de (1H-benzotriazol-1-yloxy)-diméthylamino) -N,N'-diméthylméthaniminium; 1.69 g; 4.45 mmol). Après 1 h, du *trans*-4-aminocyclohexanol (680 mg ; 4.45 mmol) a été rajouté et le mélange réactif est agité pendant 18 h à 20°C. Soixante millilitres d'eau (60 mL) sont ajoutés et le précipité a été filtré et lavé deux fois avec 60 mL d'eau pour donner 1.4 g de composé solide violet qui est utilisé tel que dans l'étape d'hydrolyse.

### Etape 2 (Procédure type de l'hydrolyse) : conversion de l'ester méthylique de C19 en C19

Les réactions chimiques ont été réalisées sous argon et protégées de la lumière par du papier d'aluminium. Les produits sont stockés à 4°C. Le 1.4 g de mèthyl ester de C-21 sont dissous dans 10 mL de tétrahydrofurane (THF) et 10 mL de MeOH (solution rouge sombre). Cinq équivalents de soude (NaOH, 10N ; 1.43 mL) sont ajoutés et le mélange réactionnel est agité pendant 48 h à température ambiante. En HPLC-MS, on observe un seul pic et l'absence de matériel de départ. De l'acide chlorhydrique (HCl 12N ; 1.18 mL) a été ajouté. Le précipité obtenu est dilué avec 60 mL d'eau. La solution est centrifugée et le surnageant aqueux est éliminé. Le culot solide est ensuite repris et mélangé avec de l'eau (30 mL) puis centrifugé à nouveau et le surnageant est éliminé. Cette étape est répétée deux fois jusqu'à l'obtention d'une pâte solide de couleur orange (qui forme une suspension en présence d'eau). La pâte solide est reprise dans 50 mL d'eau pour donner une suspension orange, congelée et directement lyophilisée pour donner une poudre orange (1.02 g).

### Analyses du composé C19

LC-MS : m/z = 478.2 (MH⁺) pureté UV à 460 nm = 97.3%.

RMN ¹H (500 MHz, DMSO-*d₆*) - δ 47.82 (s, 1H), 4.51 (m, 1H), 3.58.-3.53 (m, 1H), 3.40-3.36 (m, 1H), 1.83-1.75 (m,4H), 1.26-1.15(m, 4H).

### CASCADE DE SCREENING ET CARACTERISATION DES EFFETS BIOLOGIQUES DES COMPOSES CHIMIQUES (C) DERIVES DE NORBIXINE.

Le développement du test de criblage a été initié à partir des travaux de la littérature et basé sur les caractéristiques de la pathologie de la DMLA sèche. Au niveau physiopathologique, cette maladie se caractérise par une perte progressive de la vision induite suite à la dégénérescence des photorécepteurs et des cellules de l'EPR. Les cellules de l'EPR jouent un rôle crucial pour la survie et le bon fonctionnement des photorécepteurs en leurs apportant les nutriments nécessaires, en participant au cycle visuel et en évacuant les débris issus des segments externes des photorécepteurs et qui résultent de ce cycle. Il est important de cribler les médicaments en développement sur leur capacité à cibler préférentiellement l'œil tout en évitant une administration intraoculaire traumatisante pour les patients et présentant des risques d'infections locales. Pour ce faire, une étude pharmacocinétique des composés chimiques (C) dérivés de norbixine objet de la présente invention au niveau plasmatique et oculaire a été réalisée afin de sélectionner les composés chimiques ayant une AUC (de la terminologie anglo-saxonne *« area under the curve* ») oculaire améliorée par rapport à la norbixine.

Outre une meilleure distribution dans le tissu-cible, la sélection de nouveaux composés doit également se baser sur une bonne activité photo protectrice limitant la perte des cellules de l'EPR et ainsi réduisant la dégénérescence rétinienne observée au cours de la DMLA et d'autres maladies dégénératives comme la Rétinite pigmentaire et la maladie de Stargardt. Au niveau cellulaire, sur des cultures de cellules l'EPR issues de rétines de porcs, Fontaine et al. (2016) ont montré qu'un traitement par la norbixine (BIO201) protège les cellules de l'EPR contre l'apoptose suite à une illumination en présence d'A2E (80% de survie 24h après l'exposition). Ce même test de criblage en fonction du pourcentage de photoprotection a été utilisé afin de déterminer sa modulation par et les composés chimiques dérivés de norbixine (C) objet de la présente invention en comparaison avec l'effet photo-protecteur de la norbixine et de caractériser ces modulations d'un point de vue statistique.

### Protocoles

### Etude pharmacocinétique par voie orale des molécules chez la souris

L'étude pharmacocinétique des composés chimiques (C) suite à leur administration par voie orale a été réalisée en utilisant des souris C57BL/6 (Janvier, 53940 Le Genest Saint Isle, France). Les composés chimiques dérivés de norbixine (C) ont été administrés à une dose de 50 mg/kg de poids corporel. Après administration, le sang a été prélevé au niveau de la queue à t = 0.25 h ; 0.5 h ; 1 h ; 3 h ; 6 h et 8 h. Les échantillons de sang ont été centrifugés et les plasmas prélevés. Le dosage des échantillons de plasma a permis la détermination des paramètres pharmacocinétiques, à savoir le Cₘₐₓ, qui correspond à la concentration maximale observée après l'administration de la molécule, le Tₘₐₓ qui est le temps requis pour atteindre la concentration maximale après administration de la molécule et l'AUC : l'aire sous la courbe qui correspond à l'exposition plasmatique (figure 2).

En parallèle, les concentrations oculaires des composés chimiques dérivés de norbixine (C) ont été dosées de la façon suivante (figure 1) : les deux yeux de chaque souris ont été prélevés dans des tubes Precellys et stockés à -80°C jusqu'au moment du dosage. Les yeux sont ensuite broyés dans les tubes Precellys avec un homogénéiseur de paillasse, Fast-prep (Fischer Scientific, Hampton, Etats-Unis) dans un mélange de solvants organiques, dans premier temps avec 500 µL de chloroforme/méthanol (1/1, v/v) puis 500 µL de chloroforme/dichlorométhane (1/1, v/v). Les surnageants sont récupérés à chaque étape et transférés dans une plaque 96-puits de 2 mL.

Pour la quantification des composés chimiques C, une courbe de calibration est effectuée avec 8 standards (5 à 5000 ng/mL) dans les mêmes mélanges de solvants organiques et transférés (100 µL) dans la plaque 96-puits de 2 mL.

Les surnageants et standards sont évaporés dans un EZ2 (Genevac, Ipswich, Royaume-Uni), sans chauffage, puis repris avec 100 µL de DMSO/méthanol (20:80, v/v) avant d'être transférés dans une plaque 96 puits de 200 µL.

L'analyse LC-MSMS est effectuée avec une chaîne HPLC 1200 Infinity (Agilent Technologies, Santa-Clara, Etats-Unis), un détecteur UV et un spectromètre de masse QQQ6420 (Agilent Technologies Santa-Clara, Etats-Unis). Le volume d'injection est 5 µL. Les composés chimiques C sont élués sur une colonne de phase inverse C18 (2.1*50 mm, particules 3 µm ; Ace-C18-Excel, AIT) avec un gradient d'acétonitrile et eau (contenant 0.1 % acide formique) et un débit de 0.3 mL/min. Les conditions du gradient peuvent évoluer en fonction du composé chimique C analysé. Le détecteur UV analyse à 460 nm et le spectromètre de masse analyse en Mode MRM - Positif.

### Photoprotection des cellules de l'EPR

### Tests in vitro de photoprotection par les différents composés chimiques C des cellules de l'EPR illuminées en présence d'A2E

Le test *in vitro* précédemment décrit et destiné à étudier l'effet photo-protecteur de la norbixine a été utilisé afin de quantifier les effets photo-protecteurs des divers composés chimiques dérivés de norbixine (C) sur les cellules de l'EPR illuminées à la lumière bleue en présence d'A2E (Figure 3). L'effet photo-protecteur des molécules a été évalué dans un modèle cellulaire de phototoxicité induite par traitement par A2E suivi d'une illumination en lumière bleue. Par rayonnements bleus on entend les rayonnements correspondant à la bande bleue du spectre de la lumière visible, soit de longueur d'onde comprise entre 435 et 490 nm. Ce modèle utilise des cultures primaires d'EPR de porc adulte. La survie cellulaire est quantifiée grâce à un test de viabilité cellulaire. On ajoute à - 48 h les composés à tester (en solution à 5 mM dans le DMSO) pour obtenir des concentrations finales de 1 à 20 µM) puis à -19 h de l'A2E (concentration finale 30 µM) et on illumine les cellules (temps 0 h). On mesure 24 h après la survie des cellules. L'acquisition des images, ainsi que leurs traitements, sont réalisés à l'aide d'un microscope à fluorescence piloté par le logiciel Metamorph et d'un programme de quantification dédié. Les expériences sont réalisées sur des microplaques de 96 puits en quadruplicate et l'expérience est reproduite au minimum quatre fois. Les résultats sont exprimés sous forme d'un ratio représentant le nombre de cellules vivantes dans les puits traités par les molécules à tester, divisé par le nombre de cellules vivantes dans les puits contrôles (traités par le milieu de dilution sans A2E). Ce test a permis précédemment de mettre en évidence l'activité photo-protectrice de la norbixine (Fontaine et al. 2016).

### Résultats

### Etude pharmacocinétique des composés chimiques C chez la souris

Le tableau 2 expose les résultats de pharmacocinétique des composés chimiques C suite à l'administration p.o. 50 mg/kg dans D-α-Tocopheryl polyethylene glycol 1000 succinate (VitE-TPGS) 20% dans tampon bicarbonate de sodium (0,1M).

**[Tableau 2]**

| | Œil | | | Plasma | | |
|---|---|---|---|---|---|---|
| | C_{Max} (ng/œil) | T_{Max(}h) | Exposition (ng.h/œil) | C_{Max} (µg/mL) | T_{Max}(h) | Exposition (µg.h/mL) |
| BIO201 | 8,6 | 0,5 | 22,4 | 34,99 | 0,25 | 100,7 |
| **C1** | 10,7 | 0,5 | 26,6 | 18,73 | 0,5 | 52,66 |
| **C2** | 41 | 0,5 | 186,5 | 34 | 0,5 | 176,2 |
| **C3** | 5,1 | 0,5 | 19,1 | 4,05 | 0,5 | 10,37 |
| **C4** | 10,2 | 0,5 | 30 | 57,07 | 1 | 208,6 |
| **C5** | 31,4 | 0,5 | 188,7 | 37,71 | 1 | 299,9 |
| **C6** | 3,9 | 0,5 | 23,1 | 3,95 | 0,5 | 10,81 |
| **C7** | 7 | 0,5 | 18,5 | 6,89 | 0,5 | 17,29 |
| **C8** | 56,8 | 1 | 472,4 | 59,82 | 0,5 | 173,74 |
| **C9** | 53,1 | 1 | 452 | 37,92 | 1 | 342,68 |
| **C10** | 64,3 | 1 | 533,2 | 30,42 | 1 | 108,1 |
| **C11** | 47,7 | 1 | 238,4 | 35,91 | 1 | 190 |
| **C12** | 50,7 | 1 | 538,4 | 29,31 | 0,5 | 186,3 |
| **C13** | 60,7 | 1 | 461,1 | 27,89 | 0,5 | 139,7 |
| **C14** | 43,2 | 1 | 322,5 | 19,02 | 0,5 | 162,2 |
| **C15** | 22,8 | 0,5 | 108,5 | 17,34 | 0,5 | 46,99 |
| **C16** | 7,4 | 0,5 | 8,7 | 10,51 | 1 | 13,23 |
| **C17** | 18 | 2 | 135,2 | 8,06 | 1 | 32,39 |
| **C18** | 27,7 | 2 | 307,8 | 61,65 | 0,5 | 948,84 |
| **C19** | 5,7 | 0,5 | 37,5 | 6,62 | 0,25 | 22,8 |
| **C20** | 80,4 | 2 | 421,9 | 35,02 | 1 | 160,4 |
| **C21** | 1,6 | 0,25 | 0,6 | 6,61 | 0,25 | 9,17 |
| **C22** | 42,1 | 2 | 358,5 | 12,83 | 0,25 | 37,25 |
| **C23** | 11 | 2 | 54,7 | 5,01 | 1 | 24,1 |
| **C24** | 96 | 1,5 | 499,1 | 48,31 | 0,5 | 141,9 |

Le tableau 3 correspond aux pourcentages de photoprotection des cellules de l'EPR par les composés chimiques C *in vitro* : il illustre le pourcentage de cellules d'EPR survivantes en présence de N-rétinyl-N-rétinylidène éthanolamine (A2E) et des différents composés chimiques C dérivés de norbixine (testés à 5, 10 ou 20 µM) ou de norbixine aux mêmes concentrations après avoir été soumises à une illumination.

**[Tableau 3]**

| | **Eye Exposure** | **Photoprotection (%)** | | | | | |
|---|---|---|---|---|---|---|---|
| | **50mg/kg p.o.** | **5µM** | | **10µM** | | **20µM** | |
| | **AUC (ng.h/eye)** | **Mean** | **SE** | **Mean** | **SE** | **Mean** | **SE** |
| **BIO201** | 22,4 | 19,2 | 6,4 | 34,1 | 6,6 | 63,6 | 4,6 |
| **C1** | 26,6 | 55,0 | 4,8 | 67,7 | 2,2 | 77,8 | 7,1 |
| **C2** | 186,5 | 33,9 | 13,7 | 22,3 | 12,9 | 49,1 | 11,2 |
| **C3** | 19,1 | 11,3 | 11,3 | 68,1 | 32,1 | 45,1 | 10,3 |
| **C4** | 30,0 | 16,4 | 14,0 | 36,0 | 17,7 | 67,2 | 16,4 |
| **C5** | 188,7 | 11,3 | 11,3 | 25,6 | 23,3 | 36,0 | 11,9 |
| **C6** | 23,1 | nd | | 60,6 | 30,3 | 19,1 | 19,1 |
| **C7** | 18,5 | 5,2 | 5,2 | 52,8 | 14,8 | nd | |
| **C8** | 472,4 | 28,6 | 8,4 | 33,4 | 8,2 | 75,2 | 6,7 |
| **C9** | 452,0 | 22,1 | 9,4 | 32,0 | 12,7 | 63,7 | 6,1 |
| **C10** | 533,2 | 15,5 | 1,1 | 27,4 | 27,4 | 80,3 | 11,4 |
| **C11** | 238,4 | 21,1 | 7,7 | 39,3 | 16,7 | 63,4 | 8,2 |
| **C12** | 538,4 | 7,7 | 3,1 | 14,3 | 7,6 | 59,6 | 4,3 |
| **C13** | 461,1 | 8,0 | 4,0 | 0,5 | 0,3 | 8,0 | 6,5 |
| **C14** | 322,5 | 3,1 | 3,1 | 46,8 | 1,2 | 79,3 | 6,0 |
| **C15** | 108,5 | 5,4 | 4,0 | 10,0 | 5,1 | 58,5 | 5,7 |
| **C16** | 8,7 | 70,2 | 6,1 | 81,7 | 1,6 | 26,8 | 5,6 |
| **C17** | 135,2 | 71,7 | 5,4 | 67,2 | 6,4 | 44,0 | 4,9 |
| **C18** | 307,8 | 27,3 | 11,7 | 43,1 | 12,4 | 63,8 | 5,9 |
| **C19** | 37,5 | 60,6 | 7,5 | 60,6 | 7,5 | 79,9 | 4,1 |
| **C20** | 421,9 | 65,6 | 5,7 | 65,6 | 5,7 | 79,0 | 5,4 |
| **C21** | 0,6 | 56,7 | 7,0 | 56,7 | 7,0 | 79,6 | 1,1 |
| **C22** | 358,5 | 64,5 | 8,2 | 72,8 | 5,7 | 66,0 | 7,9 |
| **C23** | 54,7 | 38,4 | 10,4 | 59,2 | 6,8 | 73,1 | 8,3 |
| **C24** | 499,1 | 47,0 | 15,0 | 69,0 | 13,0 | 84,0 | 13,6 |

### BIBLIOGRAPHIE

AREDS Report No. 8. 2001. A randomized, placebo-controlled, clinical trial of high-dose supplementation with vitamins C and E, beta carotène, and zinc for age-related macular degeneration and vision loss. Arch Ophthalmol, 119: 1417-1436.

Bhosale P, Serban B, Bernstein PS. 2009. Retinal carotenoids can attenuate formation of A2E in the retinal pigment epithelium. Arch Biochem Biophys, 483: 175-181.

Elliott JG, Williams NS. 2012. Nutrients in the battle against age-related eye diseases. American Optométrie Association. doi:10.1016/j.optm.2011. 11.006

Fontaine V, Lafont R, Sahel JA, Veillet S. 2011. Utilisation de composés et composition pour le traitement de la dégénérescence maculaire liée à l'âge (DMLA). Demande FR 25506 (déposée le 14 mai 2011).

Fontaine V, Monteiro E, Brazhnikova E, Lesage L, Balducci C, Guibout L, Feraille L, Elena PP, Sahel JA, Veillet S, Lafont R. 2016. Norbixin protects retinal pigmented epithelium and photoreceptors against A2E-mediated phototoxicity in vitro and in vivo. PLoS ONE (2016): DOI:10.1371/journal.pone.0167793.

Laabich A, Vissvesvaran GP, Lieu KL, Murata K, McGinn TE, Manmoto CC, Sinclair JR, Karliga I, Leung DW, Fawzi A, Kubota R. 2006. Protective effect of crocin against blue light- and white light-mediated photoreceptor cell death in bovine and primate retinal primary cell culture. Invest Ophthalmol Vis Sci, 47: 3156-3163.

Lafont R, Veillet S, Sahel JA, Fontaine V, Elena PP. 2015. Composition pour la protection des cellules de l'épithélium pigmentaire rétinien. Demande FR 30891 (déposée le 30/04/2015).

Parisi V, Tedeschi M, Gallinaro G, Varano M, Saviano S, Piermarocchi S. 2008. Carotenoids and antioxidants in age-related maculopathy Italian study: multifocal electroretinogram modifications after 1 year. Ophthalmology, 115(2): 324-333.

Pinazo-Durán MD, Gómez-Ulla F, Arias L, Araiz J, Casaroli-Marano R, Gallego-Pinazo R, García-Medina JJ, López-Gálvez MA, Manzanaq L, Salas A, Zapara M, Diaz-Llopis M, García-Layana A. 2014. Do nutritional suppléments have a rôle in age macular degeneration prévention? J Ophthalmology, article ID 901686.

Smith W, Assink J, Klein R, Mitchell P, Klaver CCW, Klein BEK, Hofman A, Jensen S, Wang JJ, de Jong PTVM. 2001. Risk factors for age-related macular degeneration. Pooled findings from three continents. Ophthalmology 108: 697-704.

Sparrow JR, Cai B. 2001. Blue light-induced apoptosis of A2E-containing RPE: involvement of caspase-3 and protection by Bcl-2. Invest Ophthalmol Vis Sci, 42: 1356-1362.

Subczynski WK, Wisniewska A, Widomska J. 2010. Location of macular pigments in the most vulnerable régions of photoreceptor outer-segment membranes. Arch Biochem Biophys, 504: 61-66.

Tsuruma K, Shimazaki H, Nakashima K, Yamauchi M, Sugitani S, Shimazawa M, Iinuma M, Hara H. 2012. Annatto prevents retinal degeneration induced by endoplasmic reticulum stress in vitro and in vivo. Mol Nutr Food Res, 56: 713-724.

Veillet S, Lafont R, Dioh W. 2009. Cosmetic composition for protection from the sun containing urucum extract. Priority Application FR2009-54354 A (25 juin 2009), Application no FR 2009-54354, WO 2010-FR51323.

Yamauchi M, Tsuruma K, Imai S, Nakanishi T, Umigai N, Shimazawa M, Hara H. 2011. Crocetin prevents retinal degeneration induced by oxidative stress and endoplasmic reticulum stress via inhibition of caspase activity. Mol Cell Pharmacol, 650: 110-119.

## Revendications

1. Composé chimique de formule générale (I) suivante : 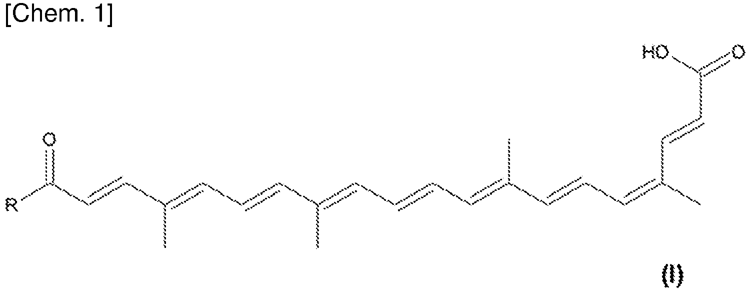
où COR est un amide secondaire ou tertiaire, tel que -R est choisi parmi :
• **-M ;** -NH-(CH₂)ₙ-**M** et -NH-(CH₂)ₙ-C(CH₃)(CH₃)-**M**, -**M** étant choisi parmi
a) 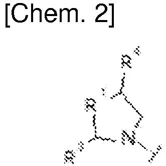
b) 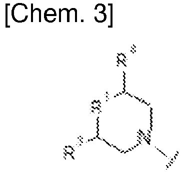
c) 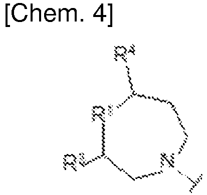 dans lesquels,
- R¹ est choisi parmi un atome d'oxygène, un atome de soufre, un groupement >CH₂, >CH-O-(CH₂)ₙ-CH₃, >CH-(CH₂)ₙ-O-(CH₂)ₙ-CH₃, >CH-(CH₂)ₙ-OH, >CH-COOH, >C(OH)phényle ou >NH ;
- R³ est choisi parmi un atome d'hydrogène, un groupement alkyle en Ci-Ce, -OH ou -O-alkyle en C₁-C₆ ;
- R⁴ est choisi parmi un atome d'hydrogène, un groupement alkyle en C₁-C₆, -OH ou -O-alkyle en C₁-C₆ ;
- n est un nombre entier compris entre 0 et 6 ;
• -NH-(CH₂)ₙ-**W**, **W** étant un atome d'hydrogène, ou un groupement -OH, un groupement -O-(CH₂)ₙ-CH₃, ou un groupement choisi parmi
i) 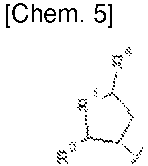
ii) 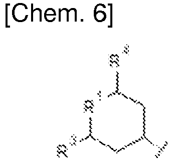
iii) 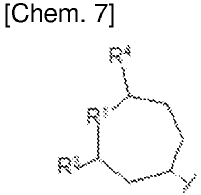
iv) 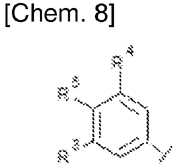
v) 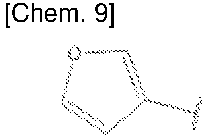 dans lesquels,
- R¹ est choisi parmi un atome d'oxygène, un atome de soufre, un groupement >CH₂, >CH-O-(CH₂)ₙ-CH₃, >CH-(CH₂)ₙ-OH, >CH-COOH, >C(OH)phényle ou >NH ;
- R³ est choisi parmi un atome d'hydrogène, un groupement alkyle en Ci-Ce, -OH ou -O-alkyle en Ci-Ce ;
- R⁴ est choisi parmi un atome d'hydrogène, un groupement alkyle en Ci-Ce, -OH ou -O-alkyle en Ci-Ce ;
- R⁵ est choisi parmi un groupement -CH₃, -OH, -O-(CH₂)ₙ-CH₃,-(CH₂)ₙ-OH ou -COOH ;
- n est un nombre entier compris entre 0 et 6 ;
ainsi que les sels pharmaceutiquement acceptables dudit composé chimique.

2. Composé chimique de formule générale **(I)** selon la revendication 1, choisi parmi les composés chimiques suivants :
- 1-[2-méthoxyéthanamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[1,4-oxazinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[pipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[2-hydoxyéthanamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1[1,4-oxazépanamido](2*E*,4*E*,6*E*,8*E*,10*E*,12*E*,14*E*,16*Z*,18*E*)-4,8,13,17-tétramethylicosa-2,4,6,8,10,12,14,16,18-nonaènedioate ;
- 1-thiomorpholinamido-(2*E*,4*E*,6*E*,8*E*,10*E*,12*E*,14*E*,16*Z*,18*E*)-4,8,13,17-tétramethylicosa-2,4,6,8,10,12,14,16,18-nonaènedioate ;
- 1-pyrrolidinamido-(2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[2-morpholinopropanamido](2*E*,4*E*,6*E*,8*E*,10*E*,12*E*,14*E*,16*Z*,18*E*)-4,8,13,17-tétramethylicosa-2,4,6,8,10,12,14,16,18-nonaènedioate ;
- 1-[(S)-3-hydroxypyrrolidinamido](2*E*,4*E*,6*E*,8*E*,10*E*,12*E*,14*E*,16*Z*,18*E*)-4,8,13,17-tétramethylicosa-2,4,6,8,10,12,14,16,18-nonaènedioate ;
- 1-[2-morpholinoethanamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[(R)-3-hydroxypyrrolidinamido](2*E*,4*E,*6*E*,8*E*,10*E*,12*E*,14*E*,16*Z*,18*E*)-4,8,13,17-tétraméthylicosa-2,4,6,8,10,12,14,16,18-nonaènedioate ;
- 1-[4-hydroxypipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[2-méthyl-2-(4-morpholinyl)propylamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[4-hydroxyméthylpipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[(Z)-2,6-diméthylmorpholinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[4-hydroxyphénylamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[4-benzyl-4-hydroxypipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[4-carboxypipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[(E)-4-hydroxycyclohexamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[3-méthoxypipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[4-méthoxypipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[2-(2-furyl)éthanamido](2*E*,4*E*,6*E*,8*E*,10*E,*12*E*,14*E*,16*Z,*18*E*)-4,8,13,17-tétraméthylicosa-2,4,6,8,10,12,14,16,18-nonaènedioate ;
- 1-[4-n-propoxypipéridinamido](2*E*,4*E*,6*E*,8*E*,10*E*,12*E*,14*E*,16*Z*,18*E*)-4,8,13,17-tétraméthylicosa-2,4,6,8,10,12,14,16,18-nonaènedioate ;
- 1-[4-éthylméthoxypipéridinamido](2*E*,4*E*,6*E*,8*E*,10*E*,12*E*,14*E*,16*Z,*18*E*)-4,8,13,17-tétraméthylicosa-2,4,6,8,10,12,14,16,18-nonaènedioate.

3. Composition comportant au moins un composé chimique objet de la présente invention selon l'une quelconque des revendications 1 ou 2.

4. Composition selon la revendication 3 comprenant au moins un excipient.

5. Composition selon l'une quelconque des revendications 3 à 4 comprenant un support sous une forme adaptée pour être ingéré, injecté dans l'œil ou injecté dans le sang.

6. Composé chimique de formule générale **(I)** selon l'une quelconque des revendications 1 à 2 ou composition selon l'une quelconque des revendications 3 à 5, pour son utilisation en tant que médicament.

7. Composé chimique de formule générale **(I)** selon l'une quelconque des revendications 1 à 2 ou composition selon l'une quelconque des revendications 3 à 5, pour son utilisation pour la photoprotection des cellules de l'épithélium pigmentaire rétinien chez les mammifères.

8. Composé chimique de formule générale **(I)** selon l'une quelconque des revendications 1 à 2 ou composition selon l'une quelconque des revendications 3 à 5, pour son utilisation pour le traitement et/ou la prévention des dommages à la rétine des mammifères causés par l'exposition aux rayonnements bleus correspondant à la bande bleue du spectre de la lumière visible, de longueur d'onde comprise entre 435 nm et 490 nm.

9. Composé chimique de formule générale **(I)** selon l'une quelconque des revendications 1 à 2 ou composition selon l'une quelconque des revendications 3 à 5, pour son utilisation pour le traitement et/ou la prévention des dommages à la rétine des mammifères causés par des dégénérescences rétiniennes.

10. Composé chimique de formule générale **(I)** selon l'une quelconque des revendications 1 à 2 ou composition selon l'une quelconque des revendications 3 à 5, pour son utilisation dans le traitement et/ou la prévention des maladies oculaires chez les mammifères.

11. Composé chimique de formule générale **(I)** selon l'une quelconque des revendications 1 à 2 ou composition selon l'une quelconque des revendications 3 à 5, pour son utilisation dans le traitement et/ou la prévention des rétinopathies chez les mammifères.

12. Composé chimique de formule générale **(I)** selon l'une quelconque des revendications 1 à 2 ou composition selon l'une quelconque des revendications 3 à 5, pour son utilisation dans le traitement et/ou la prévention de la dégénérescence maculaire liée à l'âge (DMLA) chez les mammifères.

13. Composé chimique de formule générale **(I)** selon l'une quelconque des revendications 1 à 2 ou composition selon l'une quelconque des revendications 3 à 5, pour son utilisation dans le traitement et/ou la prévention de la maladie de Stargardt et la Rétinite pigmentaire chez les mammifères.

## Patentansprüche

1. Chemische Verbindung der folgenden allgemeinen Formel **(I)** : 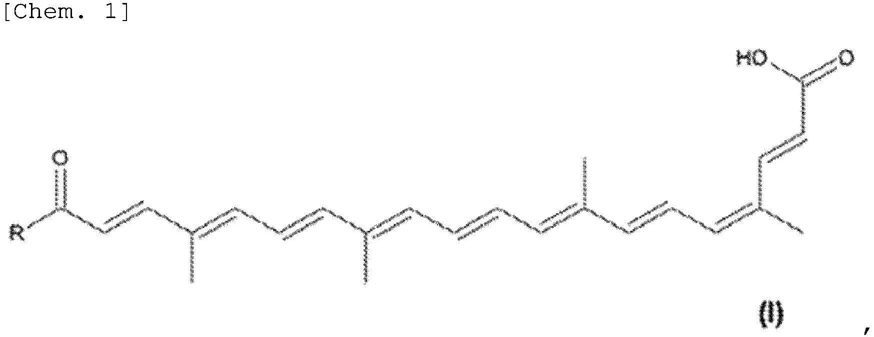
in der COR ein sekundäres oder tertiäres Amid ist, in dem -R ausgewählt ist aus:
• **-M;** -NH- (CH₂)**ₙ-M** und -NH- (CH₂) ₙ-C(CH₃)(CH₃)**-M,** wobei-**M** ausgewählt ist aus
a) 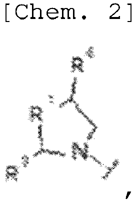
b) 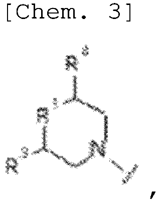
c) 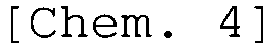 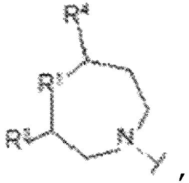 in denen
- R¹ aus einem Sauerstoffatom, einem Schwefelatom, einer >CH₂-, >CH-O-(CH₂)ₙ-CH₃-, >CH-(CH₂)ₙ-O-(CH₂)ₙ-CH₃-, >CH-(CH₂)ₙ-OH-, >CH-COOH-, >C(OH)-Phenyl- oder >NH-Gruppe ausgewählt ist;
- R³ aus einem Wasserstoffatom, einer C₁-C₆-Alkyl-, -OH-oder -O-C₁-C₆-Alkylgruppe ausgewählt ist;
- R⁴ aus einem Wasserstoffatom, einer C₁-C₆-Alkyl-, -OH-oder -O-C₁-C₆-Alkylgruppe ausgewählt ist;
• -NH-(CH₂)ₙ-**W**, wobei **W** ein Wasserstoffatom oder eine-OH-Gruppe, eine -O-(CH₂)ₙ-CH₃-Gruppe oder eine Gruppe ist, ausgewählt aus
i) 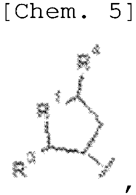
ii) 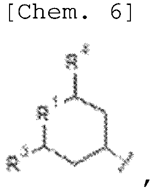
iii) 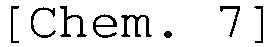 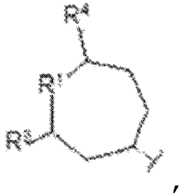
iv) 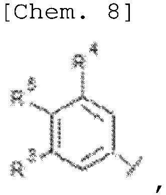
v) 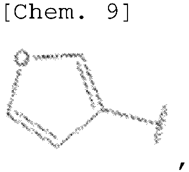 in denen
- R¹ aus einem Sauerstoffatom, einem Schwefelatom, einer >CH₂-, >CH-O-(CH₂)ₙ-CH₃-, >CH-(CH₂)ₙ-OH-, >CH-COOH-, >C(OH)-Phenyl- oder >NH-Gruppe ausgewählt ist;
- R³ aus einem Wasserstoffatom, einer C₁-C₆-Alkyl-, -OH-oder -O-C₁-C₆-Alkylgruppe ausgewählt ist;
- R⁴ aus einem Wasserstoffatom, einer C₁-C₆-Alkyl-, -OH-oder -O-C₁-C₆-Alkylgruppe ausgewählt ist;
- R⁵ aus einer -CH₃-, -OH-, -O-(CH₂)ₙ-CH₃-, -(CH₂)ₙ-OH- oder
- COOH-Gruppe ausgewählt ist;
- n eine ganze Zahl ist, die zwischen 0 und 6 liegt;
sowie die pharmazeutisch unbedenklichen Salze der chemischen Verbindung.

2. Chemische Verbindung der allgemeinen Formel **(I)** nach Anspruch 1, ausgewählt aus den folgenden chemischen Verbindungen:
- 1-[2-Methoxyethanamido](2*E*,4*E*,6*E*,8*E*,10*E*,12*E*,14*E*,16*Z*,18*E*)-4,8,13,17-tetramethylicosa-2,4,6,8,10,12,14,16,18-nonaendioat;
- 1-[1,4-Oxazinamido](2*E*,4*E*,6*E*,8*E*,10*E*,12*E*,14*E*,16*Z*,18*E*)-4,8,13,17-tetramethylicosa-2,4,6,8,10,12,14,16,18-nonaendioat;
- 1-[Piperidinamido](2*E*,4*E*,6*E*,8*E*,10*E*,12*E*,14*E*,16*Z*,18*E*)-4,8,13,17-tetramethylicosa-2,4,6,8,10,12,14,16,18-nonaendioat;
- 1-[2-Hydroxyethanamido](2*E*,4*E*,6*E*,8*E*,10*E*,12*E*,14*E*,16*Z*,18*E*) -4,8,13,17-tetramethylicosa-2,4,6,8,10,12,14,16,18-nonaendioat;
- 1-[1,4-Oxazepanamido](2*E*,4*E*,6*E*,8*E*,10*E*,12*E*,14*E*,16*Z*,18*E*)-4,8,13,17-tetramethylicosa-2,4,6,8,10,12,14,16,18-nonaendioat;
- 1-Thiomorpholinamido-(2*E*,4*E*,6*E*,8*E*,10*E*,12*E*,14*E*,16*Z*,18*E*)-4,8,13,17-tetramethylicosa-2,4,6,8,10,12,14,16,18-nonaendioat;
- 1-Pyrrolidinamido-(2*E*,4*E*,6*E*,8*E*,10*E*,12*E*,14*E*,16*Z*,18*E*)-4,8,13,17-tetramethylicosa-2,4,6,8,10,12,14,16,18-nonaendioat;
- 1-[2-Morpholinopropanamido](2*E*,4*E*,6*E*,8*E*,10*E*,12*E*,14*E*,16*Z*,18*E*) -4,8,13,17-tetramethylicosa-2,4,6,8,10,12,14,16,18-nonaendioat;
- 1-[(*S*)-3-Hydroxypyrrolidinamido] (2*E*,4*E*,6*E*,8*E*,10*E*,12*E*,14*E*,16*Z*,18*E*)-4,8,13,17-tetramethylicosa-2,4,6,8,10,12,14,16,18-nonaendioat;
- 1-[2-Morpholinoethanamido] (2*E*,4*E*,6*E*,8*E*,10*E*,12*E*,14*E*,16*Z*,18*E*)-4,8,13,17-tetramethylicosa-2,4,6,8,10,12,12,14,18-nonaendioat;
- 1-[(*R*)-3-Hydroxypyrrolidinamido] (2*E*,4*E*,6*E*,8*E*,10*E*,12*E*,14*E*,16*Z*,18*E*)-4,8,13,17-tetramethylicosa-2,4,6,8,10,12,12,14,18-nonaendioat;
- 1-[4-Hydroxypiperidinamido] (2*E*,4*E*,6*E*,8*E*,10*E*,12*E*,14*E*,16*Z*,18*E*)-4,8,13,17-tetramethylicosa-2,4,6,8,10,12,12,14,18-nonaendioat;
- 1-[2-Methyl-2-(4-morpholinyl) propylamido] (2*E*,4*E*,6*E*,8*E*,10*E*,12*E*,14*E*,16*Z*,18*E*)
- 4,8,13,17-tetramethylicosa-2,4,6,8,10,12,12,14,18-nonaendioat;
- 1-[4-Hydroxymethylpiperidinamido](2*E*,4*E*,6*E*,8*E*,10*E*,12*E*,14*E*,16*Z*, 18*E*)-4,8,13,17-tetramethylicosa-2,4,6,8,10,12,12,14,18-nonaendioat;
- 1-[(Z)-2,6-Dimethylmorpholinamido](2*E*,4*E*,6*E*,8*E*,10*E*,12*E*,14*E*,16*Z*,18*E*)-4,8,13,17-tetramethylicosa-2,4,6,8,10,12,12,14,18-nonaendioat;
- 1-[4-Hydroxyphenylamidol(2*E,*4E,6*E*,8*E*,10*E*,12*E*,14*E*,16*Z*,18*E*)-4,8,13,17-tetramethylicosa-2,4,6,8,10,12,12,14,18-nonaendioat;
- 1-[4-Benzyl-4-hydroxypiperidinamido](2*E*,4*E*,6*E*,8*E*,10*E*,12*E*,14*E*,16*Z*,18*E*)-4,8,13,17-tetramethylicosa-2,4,6,8,10,12,12,14,18-nonaendioat;
- 1-[4-Carboxypiperidinamido] (2*E*,4*E*,6*E*,8*E*,10*E*,12*E*,14*E*,16*Z*,1*8*E)-4,8,13,17-tetramethylicosa-2,4, 6,8,10,12,12,14,18-nonaendioat;
- 1-[(E)-4-Hydroxycyclohexamido] (2*E*,4*E*,6*E*,8*E*,10*E*,12*E*,14*E*,16*Z*,18*E*)-4,8,13,17-tetramethylicosa-2,4,6,8,10,12,12,14,18-nonaendioat;
- 1-[3-Methoxypiperidinamido](2*E*,4*E*,6*E*,8*E*,10*E*,12*E*,14*E*,16*Z*,18*E*)-4,8,13,17-tetramethylicosa-2,4,6,8,10,12,12,14,18-nonaendioat;
- 1-[4-Methoxypiperidinamido](2*E*,4*E*,6*E*,8*E*,10*E*,12*E*,14*E*,16*Z*,18*E*)-4,8,13,17-tetramethylicosa-2,4,6,8,10,12,12,14,18-nonaendioat;
- 1-[2-(2-Furyl) ethanamido] (2*E*,4*E*,6*E*,8*E*,10*E*,12*E*,14*E*,16*Z*,18*E*)-4,8,13,17-tetramethylicosa-2,4,6,8,10,12,12,14,18-nonaendioat;
- 1-[4-n-Propoxypiperidinamido] (2*E*,4*E*,6*E*,8*E*,10*E*,12*E*,14*E*,16*Z*,18*E*)-4,8,13,17-tetramethylicosa-2,4, 6,8,10,12,12,14,18-nonaendioat;
- 1-[4-Ethylmethoxypiperidinamido] (2*E*,4*E*,6*E*,8*E*,10*E*,12*E*,14*E*,16*Z*,1 8*E*)-4,8,13,17-tetramethylicosa-2,4,6,8,10,12,12,14,18-nonaendioat.

3. Zusammensetzung, umfassend mindestens eine chemische Verbindung zum Gegenstand der vorliegenden Erfindung nach einem der Ansprüche 1 oder 2.

4. Zusammensetzung nach Anspruch 3, umfassend mindestens einen Hilfsstoff.

5. Zusammensetzung nach einem der Ansprüche 3 bis 4, umfassend einen Träger in einer Form, die zum Ingestieren, Injizieren in das Auge oder Injizieren in das Blut angepasst ist.

6. Chemische Verbindung der allgemeinen Formel **(I)** nach einem der Ansprüche 1 bis 2 oder Zusammensetzung nach einem der Ansprüche 3 bis 5 zu deren Verwendung als Medikament.

7. Chemische Verbindung der allgemeinen Formel **(I)** nach einem der Ansprüche 1 bis 2 oder Zusammensetzung nach einem der Ansprüche 3 bis 5 zu deren Verwendung zum Lichtschutz von retinalen Pigmentepithelzellen bei Säugetieren.

8. Chemische Verbindung der allgemeinen Formel **(I)** nach einem der Ansprüche 1 bis 2 oder Zusammensetzung nach einem der Ansprüche 3 bis 5 zu deren Verwendung zur Behandlung und/oder Prävention von Schädigungen der Retina von Säugetieren, die von einer Aussetzung gegenüber blauen Strahlen verursacht werden, die dem blauen Band des Spektrums des sichtbaren Lichts entsprechen, wobei die Wellenlänge zwischen 435 nm und 490 nm liegt.

9. Chemische Verbindung der allgemeinen Formel **(I)** nach einem der Ansprüche 1 bis 2 oder Zusammensetzung nach einem der Ansprüche 3 bis 5 zu deren Verwendung zur Behandlung und/oder Prävention von Schädigungen der Retina von Säugetieren, die von Retinadegenerationen verursacht werden.

10. Chemische Verbindung der allgemeinen Formel **(I)** nach einem der Ansprüche 1 bis 2 oder Zusammensetzung nach einem der Ansprüche 3 bis 5 zu deren Verwendung zur Behandlung und/oder Prävention von Augenkrankheiten bei Säugetieren.

11. Chemische Verbindung der allgemeinen Formel **(I)** nach einem der Ansprüche 1 bis 2 oder Zusammensetzung nach einem der Ansprüche 3 bis 5 zu deren Verwendung zur Behandlung und/oder Prävention von Retinopathien bei Säugetieren.

12. Chemische Verbindung der allgemeinen Formel **(I)** nach einem der Ansprüche 1 bis 2 oder Zusammensetzung nach einem der Ansprüche 3 bis 5 zu deren Verwendung zur Behandlung und/oder Prävention einer altersbedingten Makuladegeneration (AMD) bei Säugetieren.

13. Chemische Verbindung der allgemeinen Formel **(I)** nach einem der Ansprüche 1 bis 2 oder Zusammensetzung nach einem der Ansprüche 3 bis 5 zu deren Verwendung zur Behandlung und/oder Prävention von Stargardt-Krankheit und Retinitis pigmentosa bei Säugetieren.

## Claims

1. Chemical compound having the following general formula **(I)** : 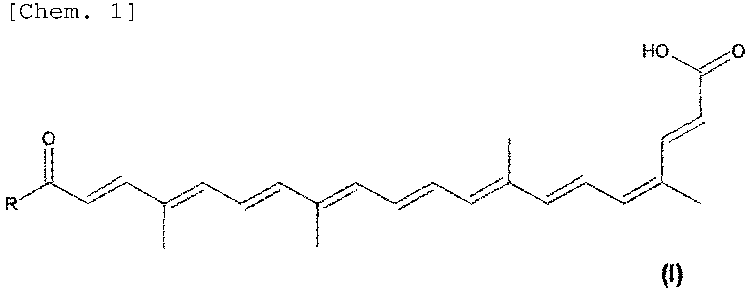
where COR is a secondary or tertiary amide, such that -R is chosen from:
• -**M**; -NH-(CH₂)ₙ-**M** and -NH-(CH₂)ₙ-C(CH₃)(CH₃)-**M**, **-M** being chosen from
a) 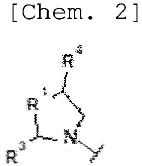
b) 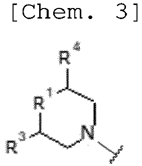
c) 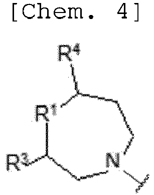 wherein,
- R¹ is chosen from an oxygen atom, a sulfur atom, a >CH₂, >CH-O-(CH₂)ₙ-CH₃, >CH-(CH₂)ₙ-O-(CH₂)ₙ-CH₃, >CH-(CH₂)ₙ-OH, >CH-COOH, >C(OH)phenyl or >NH group;
- R³ is chosen from a hydrogen atom, a C₁-C₆ alkyl, -OH or C₁-C₆ -O-alkyl group;
- R⁴ is chosen from a hydrogen atom, a C₁-C₆ alkyl, -OH or C₁-C₆ -O-alkyl group;
- n is an integer comprised between 0 and 6;
• -NH-(CH₂)ₙ-**W**, **W** being a hydrogen atom, or a -OH group, a -O-(CH₂)ₙ-CH₃ group, or a group chosen from
i) 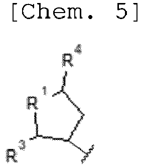
ii) 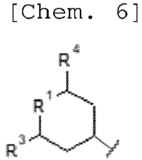
iii) 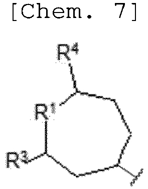
iv) 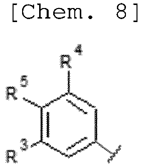
v) 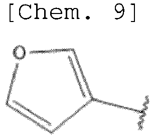 wherein,
- R¹ is chosen from an oxygen atom, a sulfur atom, a >CH₂, >CH-O-(CH₂)ₙ-CH₃, >CH-(CH₂)ₙ-OH, >CH-COOH, >C(OH)phenyl or >NH group;
- R³ is chosen from a hydrogen atom, a C₁-C₆ alkyl, -OH or C₁-C₆ -O-alkyl group;
- R⁴ is chosen from a hydrogen atom, a C₁-C₆ alkyl, -OH or C₁-C₆ -O-alkyl group;
- R⁵ is chosen from a -CH₃, -OH, -O-(CH₂)ₙ-CH₃, -(CH₂)ₙ-OH or -COOH group;
- n is an integer comprised between 0 and 6;
as well as the pharmaceutically acceptable salts of said chemical compound.

2. Chemical compound having the general formula **(I)** according to claim 1, chosen from the following chemical compounds:
- 1-[2-methoxyethanamido] (2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-[1,4-oxazinamido] (2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-[piperidinamido] (2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-[2-hydoxyethanamido] (2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1[1,4-oxazepanamido] (2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-thiomorpholinamido-(2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-pyrrolidinamido-(2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-[2-morpholinopropanamido](2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-[(*S*)-3-hydroxypyrrolidinamido](2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-[2-morpholinoethanamido](2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-[(*R*)-3-hydroxypyrrolidinamido](2*E*,4*E*,6*E*,8*E*,10*E*,12*E*,14*E*,16*Z,*18*E*)-4,8,13,17-tetramethylicosa-2,4,6,8,10,12,14,16,18-nonaenedioate;
- 1-[4-hydroxypiperidinamido](2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-[2-methyl-2-(4-morpholinyl) propylamido] (2*E*,4*E*,6*E*,8*E*,10*E*,12*E*,14*E*,16*Z*,18*E*) -4,8,13,17-tetramethylicosa-2,4,6,8,10,12,14,16,18-nonaenedioate;
- 1-[4-hydroxymethylpiperidinamido](2*E*,4*E*,6*E*,8*E*,10*E*,12*E*,14*E*,16*Z*, 18E)-4,8,13,17-tetramethylicosa-2,4,6,8,10,12,14,16,18-nonaenedioate;
- 1-[(Z)-2,6-dimethylmorpholinamido](2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-[4-hydroxyphenylamido] (2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-[4-benzyl-4-hydroxypiperidinamido](2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-[4-carboxypiperidinamido] (2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-[(E)-4-hydroxycyclohexamido] (2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-[3-methoxypiperidinamido](2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-[4-methoxypiperidinamido] (2E,4E,6E,8E,10E,12E,14E,16Z,18E)-4,8,13,17-tetramethylicosa-2,4,6,8,10,12,14,16,18-nonaenedioate;
- 1-[2-(2-furyl)ethanamido](2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-[4-n-propoxypiperidinamido](2*E*,4*E*,6*E*,8*E*,10*E*,12*E*,14*E*,16*Z*,18*E*)-4,8,13,17-tetramethylicosa-2,4,6,8,10,12,14,16,18-nonaenedioate;
- 1-[4-ethylmethoxypiperidinamido](2*E*,4*E*,6*E*,8*E*,10*E*,12*E*,14*E*,16*Z*,1 8*E*)-4,8,13,17-tetramethylicosa-2,4,6,8,10,12,14,16,18-nonaenedioate.

3. Composition including at least one chemical compound object of the present invention according to any of claims 1 or 2.

4. Composition according to claim 3 comprising at least one excipient.

5. Composition according to any of claims 3 to 4 comprising a support in a form adapted to be ingested, injected into the eye or injected into the blood.

6. Chemical compound having the general formula **(I)** according to any of claims 1 to 2 or composition according to any of claims 3 to 5, for the use thereof as a drug.

7. Chemical compound having the general formula **(I)** according to any of claims 1 to 2 or composition according to any of claims 3 to 5, for the use thereof for the photoprotection of the cells of the retinal pigment epithelium in mammals.

8. Chemical compound having the general formula **(I)** according to any of claims 1 to 2 or composition according to any of claims 3 to 5, for the use thereof for the treatment and/or the prevention of damage to the retina of mammals caused by exposure to blue light corresponding to the blue band of the visible light spectrum, of a wavelength comprised between 435 nm and 490 nm.

9. Chemical compound having the general formula **(I)** according to any of claims 1 to 2 or composition according to any of claims 3 to 5, for the use thereof for the treatment and/or the prevention of damage to the retina of mammals caused by retinal degenerations.

10. Chemical compound having the general formula **(I)** according to any of claims 1 to 2 or composition according to any of claims 3 to 5, for the use thereof in the treatment and/or the prevention of eye diseases in mammals.

11. Chemical compound having the general formula **(I)** according to any of claims 1 to 2 or composition according to any of claims 3 to 5, for the use thereof in the treatment and/or the prevention of retinopathies in mammals.

12. Chemical compound having the general formula **(I)** according to any of claims 1 to 2 or composition according to any of claims 3 to 5, for the use thereof in the treatment and/or the prevention of age-related macular degeneration (AMD) in mammals.

13. Chemical compound having the general formula **(I)** according to any of claims 1 to 2 or composition according to any of claims 3 to 5, for the use thereof in the treatment and/or the prevention of Stargardt's disease and Retinitis pigmentosa in mammals.
